# FASCICULE DE BREVET EUROPEEN

(11) **EP 1 089 678 B1**
(45) Date de publication et mention de la délivrance du brevet: **26.03.2003**
(21) Numéro de dépôt: 99925083.0
(22) Date de dépôt: 15.06.1999
(51) Int. Cl.: A61F 5/01

(54) **ORTHESE DU MEMBRE INFERIEUR**
ORTHESE FÜR DIE UNTEREN GLIEDMASSEN
ORTHOTIC DEVICE FOR A LOWER LIMB

(30) Priorité: 22.06.1998 FR 9807820
(43) Date de publication de la demande: 11.04.2001
(73) Titulaire: Chignon, Jean-Jacques, 33600 Pessac (FR)
(72) Inventeur: Chignon, Jean-Jacques, 33600 Pessac (FR)
(74) Mandataire: Verdier, Louis
(86) Numéro de dépôt international: FR9901412
(87) Numéro de publication internationale: WO99066868

(56) Documents cités:
- WO-A-95/34257
- US-A- 4 751 920
- US-A- 5 007 415
- US-A- 5 018 514
- US-A- 5 242 379

## Description

La présente invention concerne une orthèse du membre inférieur et plus particulièrement une orthèse qui permet la marche et qui permet par ailleurs une liberté totale du pied.

On connaît des orthèses du membre inférieur qui sont des orthèses rigides qui maintiennent en permanence le genou dans la même position en extension. Dans ce cas, la marche et la station debout sont autorisées mais il n'est possible de s'asseoir qu'après le retrait de l'orthèse.

On connaît aussi des orthèses qui sont articulées au niveau du genou et qui sont munies d'un verrou permettant de bloquer le genou dans la position d'extension de manière à permettre la station debout ou la marche avec la jambe raide. Il est possible de s'asseoir tout en gardant l'orthèse en place sur la jambe par déverrouillage du verrou.

Il existe d'autres orthèses comprenant un cuissard et une jambière articulés entre eux au niveau du genou. Elles sont munies de moyens de rappel élastiques qui permettent de ramener la jambe dans sa position d'extension. Ces moyens de rappel élastiques permettent au porteur d'avoir une marche plus souple que dans le cas des orthèses rigides, mais du fait que ces moyens élastiques sont dirigés dans le sens de l'extension, il n'est possible de s'asseoir que lorsque le porteur les enlève ou les détache.

Le document FR-A-2 599 246 décrit une orthèse articulée au niveau du genou qui permet au porteur de se tenir debout, de marcher sans aucune manipulation, du fait que le genou est assisté élastiquement à l'extension et à la flexion.

La demande de brevet français n° 94 07 694 décrit une orthèse du membre inférieur comprenant un cuissard et une jambière articulés entre eux au niveau du genou, l'articulation de la jambière sur le cuissard étant formée par un ensemble articulé comportant au moins une paire de biellettes disposée latéralement au genou sur au moins un côté. La paire de biellettes comprend une biellette antérieure et une biellette postérieure qui sont chacune articulée d'une part sur le cuissard et d'autre part sur la jambière, les intersections des lignes correspondant à chaque paire de biellettes définissant un axe instantané de rotation. Un tenseur est agencé latéralement sur au moins un côté entre un point d'ancrage solidaire du cuissard et un point d'ancrage solidaire de la jambière.

Le document WO-A-95/34257 décrit une structure de maintien pour l'articulation de la cheville.

Les dispositifs connus, au niveau de la jonction du pied avec la jambe, sont soit rigides, soit souples. Lorsqu'ils sont rigides, ils permettent la marche mais du fait de leur rigidité, ils ne permettent pas une souplesse de l'appui unipodal pour permettre par exemple la conduite d'un véhicule automobile. Lorsqu'ils sont souples, par exemple, lorsqu'ils comportent des releveurs élastiques tels qu'une corde à piano, un ressort à compression, un caoutchouc ajouté à un appareil sur tourillons , ils n'ont pas suffisamment de rigidité pour avoir une marche sécurisante, car il n'existe pas d'effet de stabilisation du genou .

De plus, les dispositifs connus ne permettent pas une rotation de l'appui unipodal autour de l'axe vertical.

La présente invention vise donc à remédier à ces inconvénients.

Un premier but de la présente invention est de fournir une orthèse qui puisse passer d'un état rigide à un état souple, de manière à permettre alternativement la marche sécurisante et une liberté de l'appui unipodal en rotation par rapport à l'axe vertical et le contrôle des rotations lors de la marche.

Un autre but de l'invention est de fournir une orthèse qui puisse passer d'un état rigide à un état souple d'une manière simple et pratique.

L'invention concerne donc une orthèse comprenant un appui unipodal, une tige verticale rigide, une embrasse permettant la fixation de l'orthèse sur le mollet, l'appui unipodal étant relié à une extrémité de la tige verticale et l'embrasse étant reliée à l'extrémité opposée de la tige verticale. L'orthése est telle que l'appui unipodal est relié à une extrémité de la tige verticale par l'intermédiaire d'un manchon cylindrique souple permettant la rotation horizontale de l'appui unipodal par rapport à la tige verticale et la rotation verticale de l'appui unipodal par rapport à la tige verticale et qu'elle comporte une bague cylindrique venant s'emboîter à force sur le manchon élastique de manière à bloquer selon au moins une direction la rotation verticale de l'appui unipodal par rapport à la tige verticale sans bloquer la rotation horizontale de l'appui unipodal par rapport à la tige verticale.

Selon une caractéristique de l'invention, le manchon est un cylindre sans âme en matière souple comportant à chacune de ses extrémités une fixation, lesdites fixations étant indépendantes l'une de l'autre.

Selon une autre caractéristique de l'invention, le manchon comporte une base rigide sur laquelle vient s'emboîter la bague.

De manière avantageuse, le manchon et la bague ont un diamètre correspondant.

De préférence, la bague a un diamètre interne égal au diamètre externe de la base rigide aux tolérances près, de manière que la bague vienne s'emboîter sur la base.

On pourra prévoir que l'axe longitudinal de l'appui unipodal forme un angle avec l'axe de symétrie de l'embrasse de manière que l'axe vertical soit situé latéralement à l'extérieur par rapport au porteur.

Selon encore d'autres caractéristiques de l'invention, la tige verticale peut être constituée d'une lame flexible évidée en son centre pour permettre la mise en place d'un renfort de manière à augmenter ou diminuer la rigidité de la tige lorsque la bague est en position sur la base, et le manchon peut être constitué d'un caoutchouc naturel ou synthétique.

Selon un mode de réalisation, la bague est relevée pour permettre un mouvement de rotation verticale de l'appui unipodal par rapport à la tige verticale. lame flexible 12 évidée en son centre pour permettre la mise en place d'un renfort 13, de manière à augmenter ou diminuer la rigidité de la tige 3 lorsque la bague 10 est en position sur la base. La tige 3 est évidée en son centre pour permettre la mise en place d'un renfort 13. L'embrasse 4 est reliée à l'extrémité supérieure 6 de la tige verticale. L'embrasse 4 peut comporter un système d'attache non représenté tel qu'un système par bande Velcro®. L'appui unipodal 2 comporte une base 7 destinée à reposer sur le sol. Cette base peut être creuse et en matière plastique rigide. L'appui unipodal 2 est relié à l'extrémité 5 de la tige verticale 3 et l'embrasse 4 est reliée à l'extrémité 6 de la tige verticale 3.

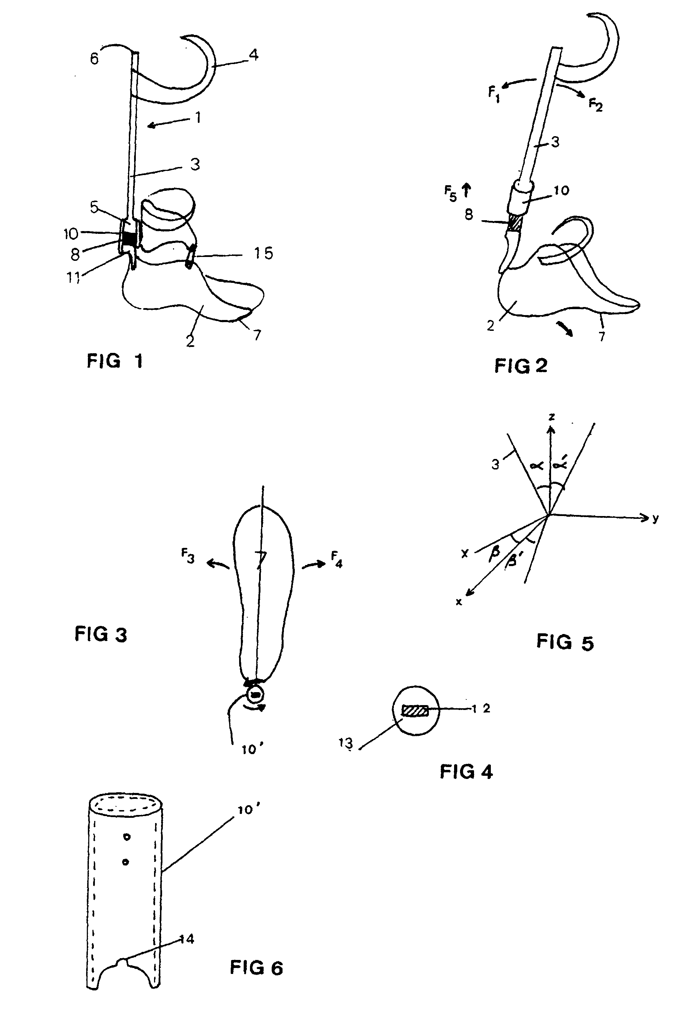

Selon l'invention, l'appui unipodal 2 est relié à l'extrémité inférieure 5 de la tige verticale 3 par l'intermédiaire d'un manchon cylindrique 8. Ce manchon 8 est souple et permet la rotation horizontale de l'appui unipodal par rapport à l'axe vertical 3. Le manchon souple 8 permet en outre la rotation verticale de l'appui unipodal 2 par rapport à la tige verticale 3. Comme on le voit sur la Figure 2, lorsque la base 7 de l'appui unipodal 2 repose sur le sol, la tige verticale 3 est mobile dans un plan vertical selon les flèches F1 et F2.

On a représenté sur la Figure 5 le plan horizontal xy représentant le sol horizontal et l'axe z représentant la verticale. Lorsque la base 7 de l'appui unipodal 2 se trouve dans le plan xy, la tige verticale 3 du dispositif peut se mouvoir entre la verticale et un angle α incliné par rapport à la verticale ou un angle α' dans le sens contraire. C'est ce qu'on appelle dans la présente invention, la rotation verticale de l'appui unipodal 2 par rapport à la tige verticale 3.

Selon l'invention, l'appui unipodal 2 lorsque sa base 7 repose sur le sol, à savoir, se trouve dans le plan xy, l'appui unipodal 2 est mobile dans un plan horizontal par rapport à la tige verticale 3 selon les flèches F3 et F4 comme on l'a représenté sur la Figure 3. Comme on le voit sur la Figure 5, l'axe longitudinal X de l'appui unipodal 2 est mobile autour de l'axe x selon un angle β et en sens contraire selon un angle β'.

Par conséquent, selon l'invention, l'orthèse permet donc un mouvement de rotation de l'appui unipodal horizontalement par torsion du manchon et verticalement de haut en bas.

L'orthèse selon la présente invention est encore telle qu'elle comporte une bague cylindrique 10 qui peut prendre deux positions différentes. La première position qui est représentée sur la Figure 1 est telle que la bague 10 vient s'emboîter sur le manchon 8 de manière à bloquer la rotation verticale de l'appui unipodal par rapport à l'axe verticale 3.

La deuxième position qui est représentée sur la Figure 2 est telle que la bague 10 est désolidarisée du manchon 8.

La bague 10 a un diamètre interne correspondant au diamètre externe du manchon 8. Le manchon 8 est un cylindre sans âme en caoutchouc comportant à chacune de ses extrémités une fixation. Ces fixations sont indépendantes l'une de l'autre. La première fixation, supérieure, relie la manchon 8 à la tige 3 et la seconde fixation, inférieure, relie l'appui unipodal 2 à la bague 10. Le manchon comporte une base rigide 11 sur laquelle vient s'emboîter la bague 10. Lorsque la bague 10 est bloquée en position emboîtée sur la base 11 du manchon 8, la rotation verticale de la tige verticale 3 et de l'appui unipodal 2 l'un par rapport à l'autre est impossible. Par contre, dans cette position, l'appui unipodal 2 peut avoir un mouvement de rotation horizontale par rapport à la tige verticale 2.

Lorsque l'on soulève la bague 10 dans le sens de la flèche F5, comme représenté sur la Figure 3, le manchon 8 est libéré, ce qui a pour effet que la bague 10 repose sur le manchon 8. La rotation verticale de l'appui unipodal 2 est alors possible.

L'emboîtement à force de la bague 10 sur le manchon 8 se fait grâce à la base 11 du manchon 8, cette base 11 ayant un diamètre externe égal au diamètre interne de la bague 10.

Ainsi, dans la position représentée sur la Figure 1, on obtient un maintien rigide selon l'axe vertical et aucun mouvement du support unipodal vers l'avant ou vers l'arrière n'est possible. Cette position permet la marche, tout en permettant les rotations.

Dans cette position telle que représentée plus particulièrement sur la Figure 3, l'appui unipodal 2 peut avoir un mouvement de rotation par rapport à la tige verticale grâce au manchon 8 élastique, ce qui permet un contrôle actif des rotations par torsion du manchon. .

Dans la seconde position représentée sur la Figure 2, la bague étant relevée, le manchon souple rend possible des mouvements de bas en haut de l'appui unipodal. L'orthèse devient alors souple et permet par exemple la conduite d'un véhicule automobile.

Le dispositif selon la présente invention permet donc à la fois la marche avec une orthèse rigide correspondant aux besoins physiologiques et se transforme en orthèse souple pour d'autres activités ne nécessitant pas une rigidité verticale.

On a représenté sur la Figure 6 une bague 10' réalisée selon une variante et légèrement modifiée par rapport à la bague 10 décrite plus haut, de manière à permettre à l'utilisateur une plus grande liberté de choix dans les mouvements rendus possibles ou interdits par l'orthèse réalisée selon la présente invention.

Selon cette variante, la bague 10' est cylindrique, l'extrémité supérieure (en position d'utilisation) de cette bague 10' étant circulaire et correspondant à une section du cylindre selon un plan perpendiculaire à l'axe du cylindre, et l'extrémité inférieure (en position d'utilisation) de cette bague 10' étant de forme générale ovale, et correspondant à une section du cylindre selon un plan ou une surface courbe (comme on l'a représenté sur la Figure 6) de direction générale non perpendiculaire à l'axe du cylindre. Il en résulte que la bague 10' présente une partie de longueur axiale (mesurée selon l'axe du cylindre) maximale et supérieure à la longueur axiale minimale d'une partie diamétralement opposée (par rapport à l'axe du cylindre). De plus, une ouverture 14 peut être pratiquée dans la bague 10', par exemple dans la partie de cette bague 10' ayant une longueur minimale.

L'orthèse, par ailleurs conforme à la description faite ci-dessus, peut être équipée d'une bague 10' au lieu de la bague 10. Dans ces conditions, la bague 10' autorise un choix supplémentaire dans les degrés de liberté autorisés en rotation verticale de l'appui unipodal de l'orthèse. En effet, selon l'orientation donnée à la partie de longueur axiale la plus grande, c'est à dire vers l'avant ou vers l'arrière (par rapport au patient), la rotation verticale de l'appui unipodal vers l'avant ou vers l'arrière respectivement sera interdite, la rotation dans l'autre sens restant autorisée.

De manière à conserver l'orientation de la bague 10' par rapport à l'appui unipodal, on peut prévoir des moyens de blocage ou de réglage en rotation de la bague 10' par rapport au manchon 8, par exemple en prévoyant une échancrure 14 dans la bague 10', cette échancrure 14 étant destinée à recevoir une vis (non représentée) coopérant par ailleurs avec un orifice taraudé pratiqué dans l'embase 11. Il est ainsi possible d'interdire ou de limiter toute possibilité de mouvement au choix soit vers l'avant, soit vers l'arrière, tout en conservant la possibilité d'effectuer des mouvements de rotation vers la droite et vers la gauche.

Lorsque le patient le désire ou en a l'autorisation, il peut obtenir la possibilité d'effectuer des mouvements vers l'avant où vers l'arrière en mettant la bague 10', le mouvement sera possible du côté ou la longueur axiale est la plus faible sans ascension de la bague 10'. Des moyens de rappel tels que des élastiques 15, assurent l'assistance du mouvement autorisé.

## Revendications

1. Orthèse comprenant un appui unipodal (2), une tige verticale rigide (3), une embrasse (4) permettant la fixation de l'orthèse sur le mollet, l'appui unipodal (2) étant relié à une extrémité (5) de la tige verticale (3) et l'embrasse (4) étant reliée à l'extrémité opposée (6) de la tige verticale (3), **caractérisée en ce que** l'appui unipodal (2) est relié à l'extrémité (5) de la tige verticale (3) par l'intermédiaire d'un manchon (8) cylindrique souple permettant la rotation horizontale de l'appui unipodal (2) par rapport à la tige verticale (3) et la rotation verticale de l'appui unipodal (2) par rapport à la tige verticale (3) et qu'elle comporte une bague (10, 10') cylindrique venant s'emboîter sur le manchon (8) élastique de manière à bloquer selon au moins une direction la rotation verticale de l'appui unipodal (2) par rapport à la tige verticale (3) sans bloquer la rotation horizontale de l'appui unipodal (2) par rapport à la tige verticale (3).

2. Orthèse selon la revendication 1, **caractérisée en ce que** le manchon (8) est un cylindre sans âme en matière souple comportant à chacune de ses extrémités une fixation, lesdites fixations étant indépendantes l'une de l'autre.

3. Orthèse selon la revendication 2, **caractérisée en ce que** le manchon (8) comporte une base rigide sur laquelle vient s'emboîter la bague (10, 10').

4. Orthèse selon la revendication 3, **caractérisée en ce que** la bague (10, 10'), a un diamètre interne correspondant au diamètre externe du manchon (8).

5. Orthèse selon l'une des revendications précédentes, **caractérisée en ce que** la bague (10, 10') a un diamètre interne égal au diamètre externe de la base (11) rigide aux tolérances près, de manière que la bague (10, 10') vienne s'emboîter sur la base (11).

6. Orthèse selon l'une des revendications précédentes, **caractérisée en ce que** l'axe longitudinal de l'appui unipodal (2) forme un angle avec l'axe de symétrie de l'embrasse de manière que l'axe vertical (3) soit situé latéralement à l'extérieur par rapport au porteur.

7. Orthèse selon l'une des revendications 2 à 5, **caractérisée en ce que** la tige verticale (3) est une lame flexible (12) évidée en son centre pour permettre la mise en place d'un renfort (13) de manière à augmenter ou diminuer la rigidité de la tige (3) lorsque la bague (10, 10') est en position sur la base.

8. Orthèse selon l'une des revendications 1 à 7, **caractérisée en ce que** le manchon (8) est constitué d'un caoutchouc naturel ou synthétique.

9. Orthèse selon l'une des revendications 1 à 8, **caractérisée en ce que** la bague (10) peut être relevée pour permettre un mouvement de rotation verticale de l'appui unipodal (2) par rapport à la tige verticale (3).

10. Orthèse selon l'une des revendications 1 à 8, **caractérisée en ce que** la bague (10') comporte une partie de longueur axiale supérieure à celle d'une partie diamétralement opposée, pour permettre, lorsque la bague (10') est maintenue en position basse, un mouvement de rotation verticale dans la direction vers laquelle est orientée la partie de longueur axiale la plus faible et interdire un mouvement de rotation verticale dans la direction vers laquelle est orientée la partie de longueur axiale la plus grande, des moyens de rappel tel que des élastiques (15), assurant l'assistance du mouvement autorisé.

## Claims

1. Orthesis comprising a single-foot support (2), a rigid vertical rod (3) and a cuff (4) allowing the orthesis to be fixed to the calf, the single-foot support (2) being attached to one end (5) of the vertical rod (3) and the cuff (4) being attached to the opposite end (6) of the vertical rod (3), **characterised in that** the single-foot support (2) is attached to the end (5) of the vertical rod (3) via the intermediary of a flexible cylindrical sleeve (8) allowing horizontal rotation of the single-foot support (2) relative to the vertical rod (3) and vertical rotation of the single-foot support (2) relative to the vertical rod (3) and that it comprises a cylindrical collar (10, 10') encasing the elastic sleeve (8) in such a way as to block in at least one direction the vertical rotation of the single-foot support (2) relative to the vertical rod (3) without blocking the horizontal rotation of the single-foot support (2) relative to the vertical rod (3).

2. Orthesis according to claim 1, **characterised in that** the sleeve (8) is an unbored cylinder of flexible material comprising a fixing at each of its ends, the said fixings being independent of each other.

3. Orthesis according to claim 2, **characterised in that** the sleeve (8) comprises a rigid base over which the collar (10, 10') fits.

4. Orthesis according to claim 3, **characterised in that** the collar (10, 10') has an internal diameter matching the external diameter of the sleeve (8).

5. Orthesis according to one of the preceding claims, **characterised in that** the collar (10, 10') has an internal diameter equal to the external diameter of the rigid base (11) with a tolerance such that the collar (10, 10') fits over the base (11).

6. Orthesis according to one of the preceding claims, **characterised in that** the longitudinal axis of the single-foot support (2) forms an angle with the axis of symmetry of the cuff such that the vertical axis (3) is located laterally to the outside relative to the wearer.

7. Orthesis according to one of claims 2 to 5, **characterised in that** the vertical rod (3) is a flexible strip (12) hollowed out at its centre to allow the insertion of a stiffener (13) such as to increase or decrease the rigidity of the rod (3) when the collar (10, 10') is in position over the base.

8. Orthesis according to one of claims 1 to 7, **characterised in that** the sleeve (8) is composed of a natural or synthetic rubber.

9. Orthesis according to one of claims 1 to 8, **characterised in that** the collar (10) may be raised to allow a vertical rotation movement of the single-foot support (2) relative to the vertical rod (3).

10. Orthesis according to one of claims 1 to 8, **characterised in that** the collar (10') comprises a part of axial length greater than that of a diametrically opposed part to allow, when the collar (10') is kept in the lowered position, a vertical rotation movement in the direction toward which the part of smallest axial length is orientated and to prevent a vertical rotation movement in the direction toward which the part of greatest axial length is orientated, with return means such as elastic bands (15) assisting the permitted movement.

## Patentansprüche

1. Prothese, umfassend eine Einsockelstütze (2), einen vertikalen steifen Schaft (3), eine Schlinge (4), die das Befestigen der Prothese an der Wade ermöglicht, wobei die Einsockelstütze (2) an ein Ende des vertikalen Schaftes (3), und die Schlinge (4) an das gegenüberliegende Ende (6) des vertikalen Schaftes (3) angeschlossen sind, **dadurch gekennzeichnet, dass** die Einsockelstütze (2) an das Ende (5) des vertikalen Schaftes mittels einer weichen zylindrischen Manschette (8) angeschlossen ist, die die horizontale Verdrehung der Einsockelstütze (2) in Bezug auf den vertikalen Schaft (3), sowie die vertikale Verdrehung der Einsockelstütze (2) in Bezug auf den vertikalen Schaft (3) erlaubt, der einen zylindrischen Ring (10, 10') umfasst, der von der elastischen Manschette (8) umschlossen ist, derart, dass er wenigstens in einer Richtung die vertikale Rotation der Einsocketstütze (2) in Bezug auf den vertikalen Schaft (3) verriegelt, ohne die horizontale Rotation der Einsockelstütze (2) in Bezug auf den vertikalen Schaft (3) zu verriegeln.

2. Prothese nach Anspruch 1, **dadurch gekennzeichnet, dass** die Manschette (8) ein Hohlzylinder aus weichem Material ist, umfassend an seinen beiden Enden eine Befestigungseinrichtung, und dass die Befestigungseinrichtungen voneinander unabhängig sind.

3. Prothese nach Anspruch 2, **dadurch gekennzeichnet, dass** die Manschette eine starre Basis umfasst, die den Ring (10,10') umschließt.

4. Prothese nach Anspruch 3, **dadurch gekennzeichnet, dass** der Ring (10, 10') eine lichte Weite aufweist, die dem Außendurchmesser der Manschette (8) entspricht.

5. Prothese nach einem der vorausgegangenen Ansprüche, **dadurch gekennzeichnet, dass** der Ring (10, 10') eine lichte Weite aufweist, die dem Außendurchmesser der starren Basis bei engen Toleranzen entspricht, derart, dass der Ring (10, 10') sich in die Basis (11) einfügt.

6. Prothese nach einem der vorausgegangenen Ansprüche, **dadurch gekennzeichnet, dass** die Längsachse der Einsockelstütze mit der Symmetrieachse der Schlinge (4) einen Winkel bildet, derart, dass die vertikalen Achse entweder seitlich oder außerhalb des Trägers liegt.

7. Prothese nach einem der Ansprüche 2 bis 5, **dadurch gekennzeichnet, dass** der vertikale Schaft (3) eine flexible Klinge (12) ist, die in ihrem Zentrum hohl ist, um eine Verstärkung (13) einzufügen, um die Steifigkeit des Schaftes (3) dann zu verringern oder zu vergrößem, wenn sich der Ring (10, 10') in seiner Position in der Basis befindet.

8. Prothese nach einem der Ansprüche 1 bis 7, **dadurch gekennzeichnet, dass** die Manschette (8) aus Naturkautschuk oder synthetischem Kautschuk besteht.

9. Prothese nach einem der Ansprüche 1 bis 8, **dadurch gekennzeichnet, dass** der Ring (10) angehoben werden kann, um eine vertikale Rotationsbewegung der Einsockelstütze (2) in Bezug auf den vertikalen Schaft (3) zu ermöglichen.

10. Prothese nach einem der Ansprüche 1 bis 8, **dadurch gekennzeichnet, dass** der Ring (10') einen Längenabschnitt aufweist, der sich über jenem eines diametral gegenüberliegenden Teiles befindet, um dann, wenn der Ring (10') in seiner unteren Position gehalten ist, eine vertikale Rotationsbewegung in jener Richtung zu erlauben, in welcher der schwächste Teil des Axialabschnittes ausgerichtet ist, und um eine vertikale Drehbewegung in jener Richtung zu unterbinden, in welcher der größte Teil des Längenabschnittes ausgerichtet ist, wobei Rückholmittel wie elastische Mittel (15) zur gewollten Bewegung beitragen.
